# EUROPÄISCHE PATENTSCHRIFT

(11) **EP 2 103 265 B1**
(45) Veröffentlichungstag und Bekanntmachung des Hinweises auf die Patenterteilung: **28.01.2015**
(21) Anmeldenummer: 09154450.2
(22) Anmeldetag: 05.03.2009
(51) Int. Cl.: A61B 17/34, A61F 9/007, A61B 17/00

(54) **Dichtung für einen Trokar**
Seal for a trocar
Joint pour un trocart

(30) Priorität: 17.03.2008 CH 3942008
(43) Veröffentlichungstag der Anmeldung: 23.09.2009
(73) Patentinhaber: Oertli-Instrumente AG, 9442 Berneck (CH)
(72) Erfinder: Di Nardo, Silvio, 9011, St. Gallen (CH); Nyffenegger, Bruno, 9436, Balgach (CH)
(74) Vertreter: Liebetanz, Michael

(56) Entgegenhaltungen:
- EP-A- 0 564 373
- EP-A- 1 886 653
- FR-A- 2 710 270
- FR-A- 2 727 849
- US-A- 5 380 288
- US-A- 5 993 471
- US-A- 6 123 689

## Beschreibung

### Technisches Gebiet der Erfindung

Die vorliegende Erfindung betrifft ein Set umfassend einen Trokar mit einer Dichtung und einem für die Ophthalmologie ausgebildeten Instrument gemäss den Merkmalen des Oberbegriffs des Anspruchs 1.

### Stand der Technik

Trokare mit Dichtungen bzw. Dichtungen für Trokare sind aus dem Stand der Technik bekannt und werden durch Fachleute in einer Vielzahl von Eingriffen am Auge eingesetzt.

Üblicherweise werden meist drei Trokare bei einem Eingriff am Auge eingesetzt. Jeder Trokar, auch als Führungsrohr bezeichnet, wird derart in das Auge eingesetzt, dass die Spitze des Trokars bis in den Glaskörperraum (Corpus vitreum) des Auges ragt, während das gegenüberliegende Ende als Anschluss bereit steht. Meist muss das Auge mit einer Infusion versorgt werden, dies geschieht über den so genannten Infusionstrokar. Zudem werden meist zwei weitere Trokare eingesetzt. Über diese weiteren Trokare können dann entsprechende Instrumente oder Beleuchtungsvorrichtungen dem Auge zugeführt werden. Diese weiteren Trokare werden auch als Instrumententrokare bzw. Beleuchtungstrokare bezeichnet.

Zwischen den Trokaren und dem eingeführten Instrument muss eine Dichtung vorgesehen sein, um ein Austreten eines Fluides bei eingesetztem Trokar zu verhindern. Beispielsweise ist die Firma DORC dazu übergegangen eine Art Kappe am Flansch des Trokars anzubringen, welche die Dichtung zwischen Trokar und Instrument bereitstellt. Eine solche Kappe ist jedoch nachteilig, da diese den Aussendurchmesser des Trokars erhöht.

Aus der US 5,865,807 ist eine weitere Dichtung bekannt, welche im Inneren eines Trokars anordbar ist. Dabei umfasst diese Dichtung zwei Dichtstellen. Dies ist bezüglich der Qualität der Dichtung vorteilhaft, hat aber den Nachteil, dass die Instrumente in Längsrichtung durch zwei Dichtungen hindurch gestossen werden müssen, wobei grössere Kräfte auf das Auge wirken.

Die EP 0 564 373 zeigt eine Dichtung für einen Trokar, wobei eine sich in Einfiihrrichtung des Instrumentes konisch erstreckende Dichtlippe angeordnet ist. Die Dichtlippe stellt dabei eine Dichtwirkung zwischen Instrument und Trokar bereit. Allerdings ist die Dichtung mit dem Nachteil behaftet, dass das Instrument sich in bzw. gegen die Einführrichtung bewegen kann, da die Dichtung und andere Elemente ausschliesslich eine kraftschlüssige Klemmverbindung zwischen Dichtung und Instrument bereitstellen.

### Darstellung der Erfindung

Ausgehend von diesem Stand der Technik liegt der Erfindung eine Aufgabe zugrunde, eine Dichtung für einen Trokar anzugeben, welche die Nachteile des Standes der Technik überwindet. Ferner soll ein Trokar angegeben werden, welcher eine einfache Herstellung einer Verbindung zwischen Instrument oder Infusionsanschluss mit dem Trokar erlaubt.

Diese Aufgabe löst ein Set umfassend einen Trokar mit einer Dichtung und einem für die Ophthalmologie ausgebildeten Instrument mit den Merkmalen des Patentanspruchs 1. Vorteilhafte Ausgestaltungen der Erfindung sind in den abhängigen Ansprüchen angegeben.

Demgemäss setzt eine solche Dichtung ein Dichtelement mit einer Dichtöffnung und eine Instrumentenöffnung ein, welche Instrumentenöffnung sich entlang einer Mittelachse auf beiden Seiten des Dichtelementes durch die Dichtung hindurch erstreckt, wobei die Dichtung weiterhin ein Klemmelement zur Herstellung einer formschlüssigen Verbindung mit einem mindestens teilweise in die Instrumentenöffnung und durch die Dichtöffnung eingeführten Instrument umfasst.

Ein vorteilhafter Trokar nach der Erfindung weist eine solche eingesetzte Dichtung auf. Durch einen aussen umlaufenden Wulst auf der Dichtung kann vorzugsweise mit einem komplementären Einstich in der Trokarinnenwand eine formschlüssige Verbindung erreicht werden, mit der auf dieses System einwirkende Einstoss- und Auszugskräfte eines Instrumentes nicht zu einer Verschiebung der Dichtung in dem Trokar führen, obwohl in sicherer Weise eine Verbindung zwischen Instrument oder Infusionsanschluss mit dem Trokar ermöglicht wird.

Weitere vorteilhafte Ausführungsformen sind in den Unteransprüchen gekennzeichnet.

### Kurze Beschreibung der Zeichnung

Bevorzugte Ausführungsformen werden im folgenden anhand der Zeichnung beispielhaft näher beschrieben. Es zeigen:
- Fig. 1: eine Schnittdarstellung eines Trokars mit einer Dichtung gemäss der Erfindung;
- Fig. 2: eine Schnittdarstellung des Trokars mit der Dichtung gemäss Figur 1 mit einem Infusionsanschluss;
- Fig. 3: eine Schnittdarstellung des Trokars mit der Dichtung gemäss Figur 1 mit einer beliebigen Instrument;
- Fig. 4a, b: eine schematische Draufsicht auf die Dichtmembran der Dichtung; und
- Fig. 5a, b: eine schematische Schnittansicht der Dichtmembran, wobei der Schnitt entlang der Mittelachse verläuft.

### Beschreibung von bevorzugten Ausführungsbeispielen

Mit Bezug zu den Zeichnungen werden mögliche Ausführungsbeispiele beschrieben. Die Zeichnungen und die Beschreibung zeigen bevorzugte Ausführungsbeispiele und sollten nicht ausgelegt werden, um die Erfindung einzuschränken, welche durch die Ansprüche definiert ist.

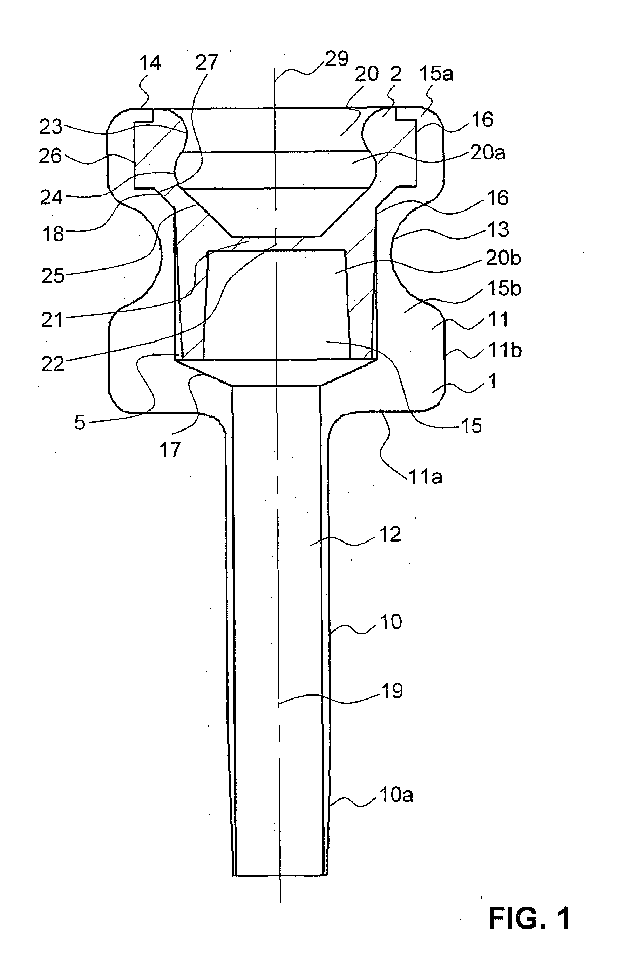

Die Figur 1 zeigt die Sclmittdarstellung eines Trokars 1 mit einer Dichtung 2. Ein solcher Trokar wird im Gebiet der Augenchirurgie eingesetzt.

Der Trokar 1 umfasst im wesentlichen eine Kanüle 10 und einen an dieser Kanüle 10 angeformten Flansch 11. Die Kanüle 10 weist einen kleineren Durchmesser als der Flansch 11 auf. Die Kanüle 10 wird durch eine Inzision im Auge in das Auge eingeführt. Dabei kommt der Flansch 11 so zu liegen, dass er mit seiner senkrecht zur Kanüle verlaufenden Oberfläche 11 a auf der Oberfläche des Auges zu liegen kommt. Der Übergang zwischen Kanüle 10 und Flansch 11 ist vorzugsweise gerundet ausgebildet.

Vorzugsweise sind sowohl Kanüle 10 als auch Flansch 11 im wesentlichen zylindrisch ausgebildet und erstrecken sich entlang einer Mittelachse 19. Die Aussenkontur der Kanüle 10 ist im unteren Bereich 10a, welcher zuerst ins Auge eingeführt wird, konisch ausgebildet, so dass ein möglichst einfaches und leichtes Einführen des Trokars 1 gewährleistet wird.

Eine zylindrische Öffnung 12 durchdringt die Kanüle 10 entlang ihrer Mittelachse 19. Im Bereich des Flansches 11 geht die zylindrische Öffnung 12 in eine Aufnahmeöffnung 15 mit grösserem Durchmesser über, welche sich vollständig durch den Flansch 11 erstreckt. Somit wird eine Öffnung 12, 15 geschaffen, welche sich durch den kompletten Trokar 1 erstreckt.

Die Aufnahmeöffnung 15 dient der Aufnahme der unten beschriebenen Dichtung 2. Hierfür weist die Aufnahmeöffnung 15 distal einen umlaufenden Einstich 16 auf, welcher sich nahe der Oberfläche 14 der Aufnahmeöffnung 15 in den Flansch 11 erstreckt. Im oberen Bereich 15a vor dem Einstich 16 weist die Aufnahmeöffnung einen grösseren Durchmesser auf, als im proximalen unteren Bereich 15b, welcher der Kanüle 10 zugewandt ist. Ferner weist die Aufnahmeöffnung 15 einen grösseren Durchmesser auf als die Öffnung 12, welche sich durch die Kanüle 10 erstreckt. Der Übergang zwischen der Aufnahmeöffnung 15 und der Öffnung 12 ist mit einer Abschrägung 17 ausgebildet. Ferner ist eine weitere Abschrägung 18 beim Übergang des Einstiches 16 zum unteren Bereich 15b vorgesehen. Der Einstich 16 kann auch anders ausgestaltet sein, sofern er, bevorzugt, einen Formschluss oder, gegebenenfalls, einen Kraftschluss mit der Dichtung 2 eingeht. Die Funktion des Einstichs 16 ist also das Halten der Dichtung 2 in dem Trokar 1.

Ferner umfasst der Trokar 1 eine aussen umlaufende Rille 13, welche hier im Bereich des Flansches 11 angeordnet ist. Dabei dringt die Rille 13 in die Mantelfläche 11b des Flansches 11 ein. Die umlaufende Rille 13 kann gut mit einer Pinzette ergriffen werden, so dass der Trokar 1 im Auge entsprechend platziert oder wieder entfernt werden kann.

Die Dichtung 2 wird ebenfalls in der Figur 1 in einer geschnittenen Darstellung gezeigt. Die Dichtung 2 umfasst eine Instrumentenöffnung 20, welche sich entlang einer Mittelachse 29 der Dichtung 2 im wesentlichen durch die gesamte Dichtung 2 erstreckt. Die Mittelachse 29 der Instrumentenöffnung 20 ist hier fluchtend mit der Mittelachse des Trokars 1. Die Instrumentenöffnung 20 dient im wesentlichen der Führung der Instrumente, welche durch den Trokar 1 hindurchgeführt werden sollen. Ferner umfasst die Dichtung 2 eine Dichtmembran 21 mit einer Dichtöffnung 22. Die Dichtmembran 21 erstreckt sich senkrecht zur Mittelachse 29 und unterteilt die Instrumentenöffnung 20 in einen oberen Abschnitt 20a und einen unteren Abschnitt 20b.

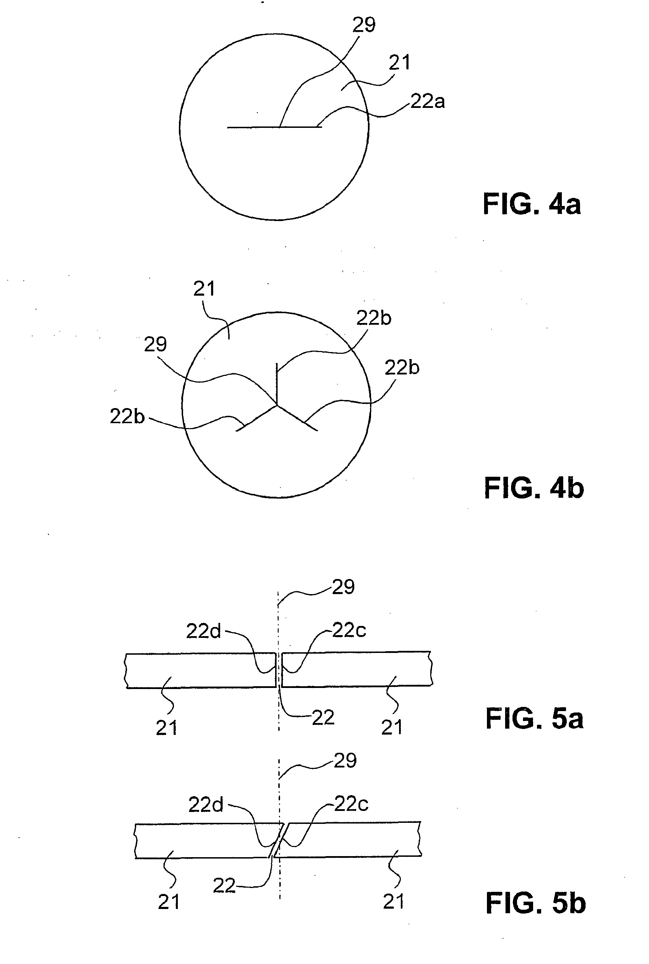

Die Dichtöffnung 22 ist dabei als bezüglich der Mittelachse 29 radial ausgeformter Schlitz 22a in der Dichtmembran 21 ausgebildet. In der Figur 4a, welche einen schematischen Ausschnitt der Dichtmembran 21 in der Draufsicht zeigt, wird der radial ausgeformte Schlitz 22a gezeigt. Dabei verläuft der Schlitz 22a in zwei radialen Strecken von der Mittelachse 29 ausgehend bis beispielsweise 2/3 des Durchmessers der Instrumentenöffnung 20. Der Schlitz und die Dichtmembran 21 sind nun dabei derart dimensioniert, dass diese einem Druck von bis 0,1 bar, oder in einem anderen Ausführungsbeispiel von 0,2 bar, welcher vom unteren Abschnitt 20b auf die Dichtmembran 21 wirkt, standhalten bzw. eine Dichtwirkung aufrechterhalten kann. Im Anwendungsfall für den Trokar 1 ist der besagte Druck ein Augeninnendruck. Das heisst mit anderen Worten, dass die Dichtmembran 21 und die Dichtöffnung 22 die durchgehende Öffnung durch den gesamten Trokar 1 auch in dem Falle abdichten kann, wenn kein Instrument durch den Trokar 1 bzw. durch die Dichtung 2 geführt wird.

In anderen Ausführungsformen, wie beispielsweise in der Figur 4b gezeigt, verlaufen drei Schlitze 22b in radialer Richtung von der Mittelachse 29. Dabei sind die Schlitze beispielsweise in einem Winkel von 120° angeordnet. Andere Anordnungen der Schlitze sind ebenfalls denkbar.

Der obere Abschnitt 20a der Instrumentenöffnung 20 umfasst ferner ein Klemmelement 23. Das Klemmelement 23 ist derart ausgebildet, dass es mit einem einzuführenden Instrument eine lösbare formschlüssige Verbindung eingehen kann, so dass eine axiale Verschiebung des Instrumentes relativ zum Trokar 1 vermieden werden kann. Das Klemmelement 23 erstreckt sich hierfür von der Oberfläche der Instrumentenöffnung 20 in die Instrumentenöffnung 20 hinein, so dass deren Durchmesser im Bereich des Klemmelementes 23 verkleinert wird. In allgemeiner Form kann gesagt werden, dass die Dichtung 2 demnach nicht nur als blosses Dichtelement, sondern auch als Befestigungselement oder Klemmelement dient.

Im vorliegenden Ausführungsbeispiel ist das Klemmelement 23 als konvexer und umlaufender Klemmwulst ausgebildet. Dabei bildet der Klemmwulst den ersten Teil des oberen Abschnittes 20a der Instrumentenöffnung 20. Nach dem Klemmwulst geht die Oberfläche der Instrumentenöffnung 20 in eine konkave Rundung 24 über. Der konkaven Rundung 24 schliesst sich eine Abschrägung 25 an, welche in die Dichtmembran 21 übergeht. Dabei verringert die Abschrägung 25 den Durchmesser der Instrumentenöffnung 20 gegen die Dichtmembran 21 hin.

Vorteilhaft ist dabei, dass das Klemmelement 23 gegenüber dem oberen Bereich 15a und dem Einstich 16 angeordnet ist, so dass beim Hineinstossen oder beim Hinausziehen eines Instrumentes radial wirkende Kräfte sicherstellen, dass es keine longitudinale Verschiebung der Dichtung 2 im Trokar 1 gibt.

In anderen Ausführungsbeispielen ist es beispielsweise denkbar, dass der Klemmwulst 23 abschnittsweise unterbrochen ist. Der obere Abschnitt 20a kann auch als Kupplungsabschnitt oder Einschnappabschnitt bezeichnet werden. Ferner ist es auch denkbar, den Klemmwulst als Schnapphalter zu bezeichnen.

Beim Einführen eines Instrumentes 3 wird das Klemmelement 23 durch Teile des Instrumentes 3 leicht verformt. Das heisst, dass bei einem feststehenden Einstich 16 der Klemmwulst 23 beim Einführen des Instrumentes leicht komprimierbar ist, so dass das Instrument einführbar ist. Das Instrument 3 weist einen zum Klemmelement 23 komplementär ausgebildeten Abschnitt auf. Das heisst, der komplementäre Abschnitt des Instrumentes 3 weist im Querschnitt gesehen, die eine Form auf, welche vom entsprechenden Abschnitt des Klemmelementes 23 gegengleich ausgebildet ist. Sobald das Instrument 3 soweit vorgeschoben wurde, dass dieses die gewünschte Einführtiefe erreicht hat, nimmt das Klemmelement 23 seine ursprüngliche Gestalt wieder ein. Der Klemmwulst 23 ragt dann in den komplementären Abschnitt ein, welcher hier eine entsprechende Vertiefung oder Rille aufweist. Durch dieses Einragen oder Einrasten kann zwischen dem Instrument 3 und der Dichtung 2 eine formschlüssige Verbindung bereitgestellt werden. Der komplementäre Abschnitt kann auch als Kupplungsabschnitt 34 bezeichnet werden.

Im allgemeinen wird unter einer formschlüssigen Verbindung eine Verbindung von zwei Elementen verstanden, wobei Teile des einen Elementes in komplementär ausgebildete Abschnitte des anderen Elementes einragen, so dass eine relative Bewegung der beiden Elemente zueinander bis zu einem gewissen Kraftaufwand verhindert werden kann.

In einer alternativen Ausführungsform könne das Klemmelement 23 auch als Rille ausgebildet sein, wobei das Instrument dann den komplementären Wulst umfasst.

Der untere Abschnitt 20b der Instrumentenöffnung 20 verläuft von der Dichtmembran 21 im wesentlichen leicht konisch, so dass der Innendurchmesser der Instrumentenöffnung 20 in Richtung der Mittelachse 29 von der Dichtmembran 21 her gesehen grösser wird. Die Instrumentenöffnung 20 in diesem unteren Abschnitt 20b kann auch als Membranhof bezeichnet werden, da die Dichtmembran 21 beim Durchführen eines Instrumentes in diesen Bereich zu liegen kommt. Die Aussenwand in diesem Abschnitt verläuft ebenfalls von der Dichtmembran her gesehen leicht konisch, dadurch entsteht zwischen der Wand der Aufnahmeöffnung 15 und der Aussenwand der Dichtung 2 ein kleiner Zwischenraum 5. Der Zwischenraum 5 hat im wesentlichen zwei Funktionen. Bei der Montage der Dichtung 2 in den Trokar 1 wird das Einführen der Dichtung 2 erleichtert, da in diesem Bereich kein Kontakt zwischen Dichtung 2 und Trokar 1 stattfindet und somit keine Reibungskräfte auftreten. Beim Einführen eines Instrumentes durch die Dichtung 2 kann sich das Dichtung nach aussen, also gegen den Trokar 1 hin, deformieren. Dadurch kann der Chirurg das Instrument mit einem geringeren axialen Kraftaufwand einführen.

Auf der Aussenseite weist die Dichtung 2 im oberen Bereich einen umlaufenden Flansch 26 auf. Der umlaufende Flansch 26 ist derart ausgebildet, dass er komplementär zu dem Einstich 16 ist und im Eingriff mit diesem steht. Ferner weist die Dichtung eine äussere Abschrägung 27 auf, welche im Eingriff mit einer Abschrägung 18 der Öffnung 15 ist. Die Verbindung zwischen Flansch 26 und Einstich 16 erlaubt eine gute Verbindung zwischen Dichtung 2 und Trokar 1. Aufgrund der Ausgestaltung können sowohl axiale als auch radiale Kräfte kompensiert werden. Mit anderen Worten kann auch gesagt werden, dass die Form der Aussenseite der Dichtung 2 im oberen Bereich 20a im wesentlichen kongruent mit der Form der Innenwand der Aufnaluneöffiung 15.

Vorzugsweise ist die Dichtung 2 aus einem Silikokunststoff gefertigt. Der Silikonkunststoff weist vorzugsweise eine Shorehärte im Bereich von Shore 70 bis Shore 80 auf. Andere Shorehärten sind aber ebenfalls denkbar. Auch können andere Kunststoffe, insbesondere thermoplastische Elastomere (TPE), für die Dichtung eingesetzt werden. Der Trokar 1 besteht aus Metall, wie beispielsweise Instrumentenstahl, Titan oder einer Titanlegierung, oder aus einem Kunststoff mit einer ausreichenden Festigkeit. Die verwendeten Materialien müssen bioverträglich sein.

Vorzugsweise wird die Dichtung 2 entlang der Mittelachse 19 in die Aufnahmeöffnung 15 eingeführt. Aufgrund der oben beschriebenen Form der Dichtung folgt eine formschlüssige Verbindung zwischen der Dichtung 2 und dem Trokar 1. In alternativen Ausführungsbeispielen ist es zudem denkbar, die Dichtung 2 einzukleben oder mittels einem Kunststoffspritzverfahren direkt in die Aufnaluneöffnung 15 einzuspritzen. Beispielsweise kann hier ein Zwei-Komponenten-Spritzgussverfahren zur Anwendung kommen. Alternativ können der Trokar 1 und die Dichtung 2 auch einstückig ausgebildet sein.

Wie gezeigt wird, ist die Dichtung 2 vollständig im Trokar 1 angeordnet. Dies ist vorteilhaft, denn dadurch wird der Durchmesser des Trokars 1 nicht, wie bei anderen Dichtungsanordnungen, vergrössert. Dadurch hat der Augenchirurg eine bessere Übersicht über das zu operierende Auge und er kann die Trokare besser handhaben.

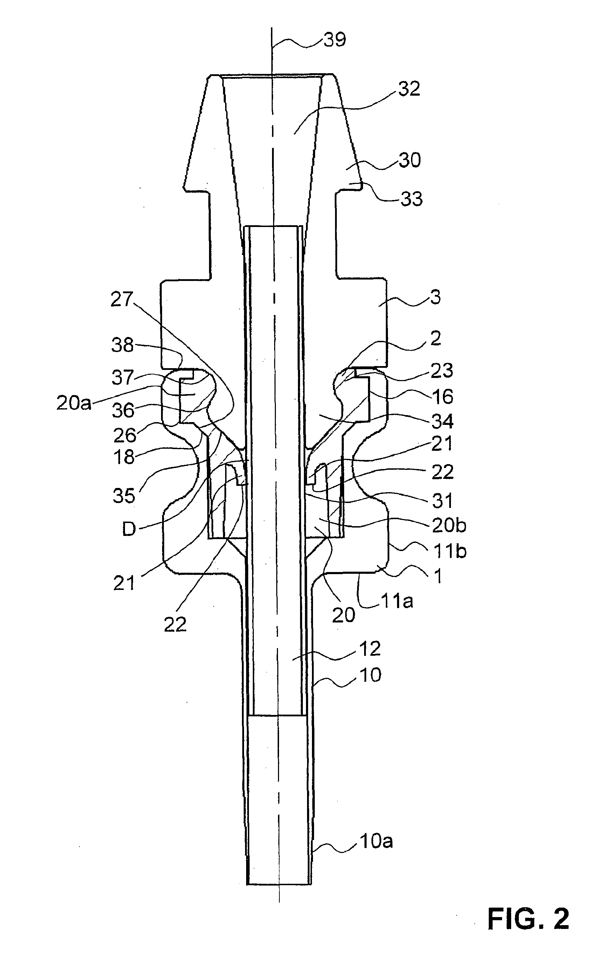

Die Figur 2 zeigt den Trokar 1 und die Dichtung 2, wobei ein Infusionsanschluss 3 in Verbindung mit dem Trokar 1 und der Dichtung 2 steht. Der Infusionsanschluss 3 umfasst hier ein Kupplungsstück 30 und ein Anschlussrohr 31.

Das Kupplungsstück 30 umfasst eine Durchgangsöffnung 32, einen Schlauchanschlussabschnitt 33 und einen Kupplungsabschnitt 34. Die Durchgangsöffnung 32 dient der Aufnahme des Anschlussrohres 31 und erstreckt sich entlang einer Mittelachse 39 vollständig durch das Kupplungsstück 30. Dabei steht das Anschlussrohr 31 in axialer Richtung über das Kupplungsstück 30 hervor.

Der Schlauchanschlussabschnitt 33 dient der Verbindung des Kupplungsstückes 30 mit einem Infusionsschlauch und ist entsprechend ausgebildet.

Der Kupplungsabschnitt 34 ist im wesentlichen komplementär zum oberen Abschnitt 20a der Instrumentenöffnung 20 ausgebildet. Der Kupplungsabschnitt 34 hat dabei im vorderen Bereich die Form eines Konus 35. Dem Konus 35 schliesst sich eine konvexe Rundung 36 an, welche in eine konkave Rundung 37 übergeht. Die konkave Rundung 37 schliesst den Kupplungsabschnitt 34 ab. Wir oben erwähnt schliesst sich dem Kupplungsabschnitt 34 der Schlauchanschlussabschnitt 33 an. Im unmittelbar an den Kupplungsabschnitt 34 anschliessenden Bereich weist der Schlauchanschlussabschnitt 33 eine senkrecht zur Mittelachse verlaufende Anschlagsfläche 38 auf.

Beim Einführen des Infusionsanschlusses 3 in den Trokar 1 bzw. in die Dichtung 2 durchdringt das Anschlussrohr 31 in einem ersten Schritt die Dichtöffnung 22 in der Dichtmembran 21. Dabei entsteht zwischen Dichtöffnung 22 und Anschlussrohr 31 ein fluiddichter Kontakt. Die Dichtstelle ist mit dem Bezugszeichen D bezeichnet. Die Dichtmembran 21 wird dabei in den Membranhof bzw. den unteren Abschnitt 20b der Instrumentenöffnung 20 verdrängt.

In einem zweiten Schritt trifft der Konus 35 auf das Klemmelement 23. Dabei wird das Klemmelement 23 in radialer Richtung komprimiert, so dass der Infusionsanschluss 3 in axialer Richtung weiter bewegt werden kann. Dabei wird der Infusionsanschluss 3 solange in axialer Richtung bewegt, bis die konkave Rundung 37 auf die Höhe des Klennnelementes 23 zu liegen kommt. Das heisst mit anderen Worten, dass das Klemmelement 23 die konkave Rundung 37 ausfüllt. Dabei entsteht zwischen der Dichtung 2 und dem Infusionsanschluss 3 eine formschlüssige Verbindung. Diese formschlüssige Verbindung kann auch als Schnappverbindung bezeichnet werden, da der Kupplungsabschnitt 34 im oberen Abschnitt 20a bzw. im Kupplungsabschnitt einschnappt bzw. einrastet. Die formschlüssige, eingerastete Verbindung ist besonders vorteilhaft, denn dadurch wird der Infusionsanschluss fest im Trokar 1 gehalten und stabilisiert. Aufgrund der kongruenten Form der beiden Kupplungsabschnitte 20a und 34 wird die Verbindung weiterhin bezüglich Querkräften, welche quer zu den Mittelachsen auftreten, zusätzlich stabilisiert.

Beim Entfernen des Infusionsanschlusses 3 muss die Zugkraft leicht erhöht werden, so dass das Klemmelement 23 wiederum entsprechend komprimiert wird. Wesentlich ist, dass die haltenden Kräfte beim Lösen vom Kupplungsabschnitt 34 gegenüber der konkaven Rundung oder umlaufenden Nut 24 kleiner sind als die Kräfte, die zwischen dem Einstich 16 und dem oberen Abschnitt 20a der Dichtung 2 wirken.

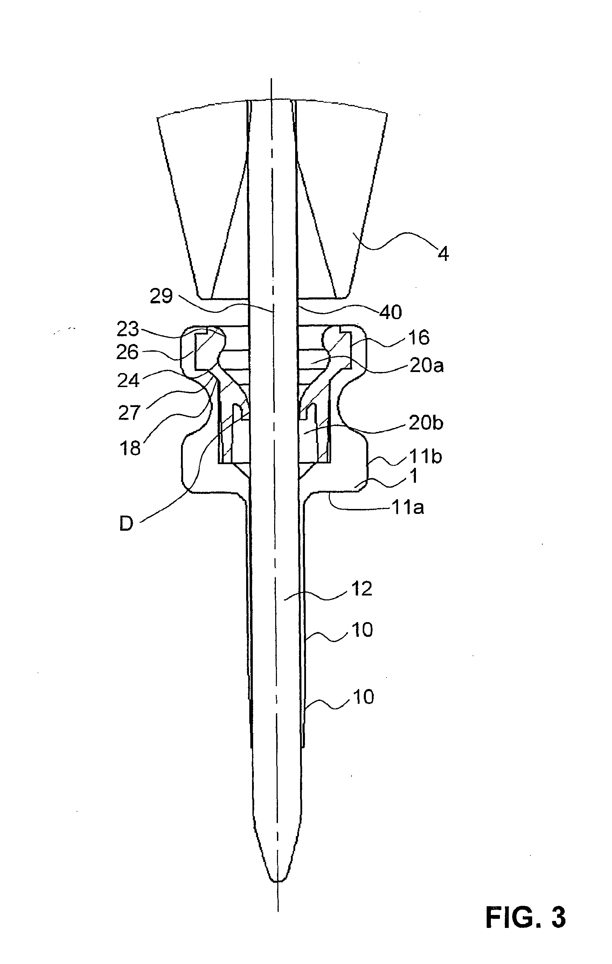

Die Figur 3 zeigt ein beliebiges nicht erfindungsgemäßes Instrument 4, welches in Verbindung mit dem Trokar 1 bzw. mit der Dichtung 2 steht. Das Instrument 4, beispielsweise eine Lichtquelle oder ein Schneidinstrument, ist hier mit einem zylindrischen Stab oder Dorn 40 dargestellt. Analog zur oben beschriebenen Verbindung wird das Instrument 4 mit dem Dorn 40 entlang seiner Mittelachse in den Trokar 1 bzw. in die Dichtung 2 eingeschoben. Dabei durchdringt der Dorn 40 analog zum Anschlussrohr 31 die Dichtmembran 21 bzw. die Dichtöffnung 22. Dabei wird ebenfalls ein fluiddichter Kontakt zwischen Dichtöffnung 22 und der Oberfläche des Dornes 40 erzielt. Zudem kann das Instrument 4 mit einer geringen Kraft entlang der Mittelachse bewegt werden, ohne dass die Dichtwirkung zwischen Dorn 40 negativ beeinflusst wird. Der Dorn 40 kann beispielsweise ein 23Gauge-Dorn sein. Alternativ kann der Dorn 40 auch ein 25Gauge-Dorn oder ein 20Gauge-Dorn sein. Das heisst, der Trokar 1 bzw. die Dichtung 2 können 20Gauge-, 23Gauge- und 25Gauge-Instrumente aufnehmen. Andere Durchmesser sind ebenfalls denkbar.

Sobald nun der Infusionsanschluss 3 oder das Instrument 4 aus dem Trokar 1 bzw. aus der Dichtung 2 entfernt wird, verschliesst sich die Dichtmembran 21 bzw. die Dichtöffnung aufgrund der elastischen Eigenschaften der Dichtmembran 21. Dies ist besonders Vorteilhaft, da keine Fluide aus dem Auge nach dem Entfernen der Instrumente 3, 4 austreten können.

Aus der Figur 3 und 5a ist ersichtlich, dass die Unterseite der abgebogenen Dichtmembran 21 flach ist und senkrecht zu den Grenzflächen der Dichtmembran 21 selber verläuft. Das heisst mit anderen Worten, dass der Schlitz oder die Schlitze der Dichtöffnung 22 im wesentlichen senkrecht zur Oberfläche der Dichtmembran 21, also parallel zur Mittelachse 19, 29 verlaufen. Die beiden Seitenwände 22c und 22d der Schlitze stehen demnach senkrecht zur Oberfläche der Dichtmembran 21.

In anderen Ausführungsbeispielen, wie in Figur 5b gezeigt, wäre es auch möglich, den oder die Schlitze der Dichtöffnung 22 in einem Winkel zur Achse 19,29 stehen. Die Seitenwände 22c und 22d stehen demnach winklig zur Mittelachse 19, 29 bzw. winklig zur Oberfläche der Dichtmembran 21.

**Bezugszeichenliste**

| | | | |
|---|---|---|---|
| 1 | Trokar | 3 | Infusionsanschluss |
| 2 | Dichtung | 4 | Instrument |
| 5 | Zwischenraum | 22a,b | Schlitze |
| 10 | Kanüle | 22c,d | Seitenwände der Schlitze |
| 10a | konischer Teil | 23 | Klemmelement |
| 11 | Flansch | 24 | konkave Rundung |
| 11a | Oberfläche | 25 | Abschrägung |
| 11b | Mantelfläche | 26 | umlaufender Flansch |
| 12 | Öffnung | 27 | Abschrägung |
| 13 | umlaufende Rille | 29 | Mittelachse |
| 14 | Oberseite Flansch | 30 | Kupplungsstück |
| 15a | oberer Bereich Aufnahmeöffnung | 31 | Anschlussrohr |
| 15b | unterer Bereich Aufnahmeöffnung | 32 | Durchgangsöffnung |
| 16 | Einstich | 33 | Schlauchanschlussabschnitt |
| 17 | Abschrägung | 34 | Kupplungsabschnitt |
| 18 | Abschrägung | 35 | Konus |
| 19 | Mittelachse | 36 | konvexe Rundung |
| 20 | Instrwnentenöffnung | 37 | konkave Rundung |
| 20a | oberer Abschnitt | 38 | Anschlagsfläche |
| 20b | unterer Abschnitt | 39 | Mittelachse |
| 21 | Dichtmembran | 40 | Dorn |
| 22 | Dichtöffnung | | |

## Patentansprüche

1. Set umfassend einen Trokar (1) mit einer Dichtung (2) und einem für die Ophthalmologie ausgebildeten Instrument (3), wobei der Trokar (1) eine Kanüle (10) mit einer Mittelachse (19), einen an dieser Kanüle (10) angeformten Flansch (11), eine Öffnung (12), welche sich entlang der Mittelachse (19) durch die Kanüle (10) erstreckt, und eine Aufnahmeöffnung (15) umfasst, welche sich entlang der Mittelachse (19) durch den Flansch (11) erstreckt, wobei die Öffnung (12) und die Aufnahmeöffnung (15) eine den Trokar vollständig durchdringende Öffnung bereitstellen; und wobei die Dichtung (2) in der Aufnahmeöffnung (15) des Trokars (1) angeordnet ist, wobei die Dichtung (2) ein Dichtelement (21) mit einer Dichtöffnung (22) und eine Instrumentenöffnung (20) umfasst, welche Instrumentenöffnung (20) sich entlang einer Mittelachse (29) auf beiden Seiten des Dichtelementes (21) durch die Dichtung (2) hindurch erstreckt, **dadurch gekennzeichnet, dass** die Dichtung (2) weiterhin ein Klemmelement (23) zur Herstellung einer formschlüssigen Verbindung mit dem mindestens teilweise in die Instrumentenöffnung (20) und durch die Dichtöffnung (22) eingeführten Instrument (3) umfasst, und dass das für die Ophthalmologie ausgebildete Instrument (3), welches durch den Trokar (1) hindurchführbar ist, einen zum Klemmelement (23) komplementären oder kongruenten Abschnitt umfasst, wobei das Klemmelement (23) und/oder das Instrument (3) derart ausgestaltet sind, dass diese während des Einschiebens des Instrumentes (3) in die Dichtung (2) komprimierbar sind, und so dass die ursprüngliche Gestalt dieser Teile beim Erreichen einer vordefinierten Einführtiefe wieder einnehmbar ist, so dass die Oberfläche des Klemmelementes (23) mit derjenigen des komplementären Abschnittes des Instrumentes in Kontakt ist, so dass eine formschlüssige Verbindung zwischen Dichtung (2) und Instrument (3) bereitgestellt wird.

2. Set nach Anspruch 1, **dadurch gekennzeichnet, dass** das Klemmelement (23) einstückig mit der Dichtung (2) ausgestaltet ist.

3. Set nach Anspruch 1 oder 2, **dadurch gekennzeichnet, dass** das Dichtelement eine Membran (21) ist, die im wesentlichen senkrecht zur Mittelachse (29) der Dichtung (2) verläuft, wobei die Dichtöffnung (22) mindestens einen Schlitz umfasst.

4. Set nach einem der vorhergehenden Ansprüche, **dadurch gekennzeichnet, dass** das Klemmelement (23) in Einführrichtung eines Instrumentes her gesehen vor dem Dichtelement (21) angeordnet ist.

5. Set nach einem der vorhergehenden Ansprüche, **dadurch gekennzeichnet, dass** das Klemmelement (23) als umlaufender Klemmwulst ausgebildet ist, welcher den Durchmesser der Instrumentenöffnung (20) verkleinert.

6. Set nach einem der vorhergehenden Ansprüche, **dadurch gekennzeichnet, dass** das Klemmelement (23) als abschnittsweise unterbrochener Klemmwulst ausgebildet ist, wobei mindestens drei Klemmwulstabschnitte gebildet werden, welche den Durchmesser der Instrumentenöffnung (20) verkleinern.

7. Set nach einem der Ansprüche 1 oder 2, **dadurch gekennzeichnet, dass** das Klemmelement (23) als vollständig umlaufende oder als abschnittsweise unterbrochene Rille ausgebildet ist.

8. Set nach einem der vorhergehenden Ansprüche, **dadurch gekennzeichnet, dass** die Dichtung (2) auf ihrer Aussenseite einen umlaufenden Flansch (26) umfasst.

9. Set nach einem der vorhergehenden Ansprüche, **dadurch gekennzeichnet, dass** die Aufnahmeöffnung (15) eine umlaufende Rille (16) umfasst, in welche ein umlaufender Flansch (26) der Dichtung (2) zur Herstellung einer formschlüssigen Verbindung zwischen Trokar (1) und Dichtung (2) eingreift.

10. Set nach einem der vorhergehenden Ansprüche, **dadurch gekennzeichnet, dass** die Dichtung (2) mittels einem Kunststoffspritzverfahren direkt in die Aufnahmeöffnung (15) des Trokars (1) eingespritzt wird.

11. Set nach einem der Ansprüche 1 bis 9, **dadurch gekennzeichnet, dass** der Trokar (1) und die Dichtung (2) aus einem Kunststoff einstückig hergestellt sind.

12. Set nach einem der vorhergehenden Ansprüche, **dadurch gekennzeichnet, dass** der komplementäre Abschnitt des Instrumentes (3) die Gestalt einer vollständig oder teilweise umlaufenden Rille aufweist, in welche der Wulst des Klemmelementes (23) einragt.

13. Set nach einem der vorhergehenden Ansprüche, **dadurch gekennzeichnet, dass** der komplementäre Abschnitt des Instrumentes (3) die Gestalt eines vollständig oder teilweise umlaufenden Wulstes aufweist, welcher durch eine am Klemmelement (23) angeordnete Rille aufnehmbar ist.

## Claims

1. Kit comprising a trocar (1) with a seal (2) and an instrument (3) which is designed for ophthalmology
wherein the trocar (1) comprises a cannula (10) with a centre axis (19), a flange (11) formed integrally on this cannula (10), an opening (12) extending along the centre axis (19) through the cannula (10), and a receiving opening (15) that extends along the centre axis (19) through the flange (11), said opening (12) and said receiving opening (15) forming an opening that runs all the way through the trocar, and wherein the seal (2) is arranged in the receiving opening (15) of the trocar (1),
wherein the seal (2) comprises a seal element (21) with a seal opening (22) and an instrument opening (20), which instrument opening (20) extends along a centre axis (29) on both sides of the seal element (21) all the way through the seal (2),
**characterized in that** the seal (2) also comprises a clamp element (23) for establishing a form-fit connection to an instrument that is inserted at least partially into the instrument opening (20) and through the seal opening (22) and **in that** the instrument (3) comprising a section that is complementary or congruent to the clamp element (23), the clamp element (23) and/or the instrument (3) being designed in such a way that they can be compressed during insertion of the instrument (3) into the seal (2) and such that the original shape of these parts can be recovered when the predefined depth of insertion is reached, such that the surface of the clamp element (23) is in contact with the surface of the complementary section of the instrument, such that a form-fit connection is established between seal (2) and instrument (3).

2. Kit according to Claim 1, **characterized in that** the clamp element (23) is designed in one piece with the seal (2).

3. Kit according to Claim 1 or 2, **characterized in that** the seal element is a membrane (21) which extends substantially perpendicular to the centre axis (29) of the seal (2), and the seal opening (22) comprises at least one slit.

4. Kit according to one of the preceding claims, **characterized in that** the clamp element (23) is arranged before the seal element (21), seen in the direction of insertion of an instrument.

5. Kit according to one of the preceding claims, **characterized in that** the clamp element (23) is designed as a circumferential clamping bead, which reduces the diameter of the instrument opening (20).

6. Kit according to one of the preceding claims, **characterized in that** the clamp element (23) is designed as a clamping bead divided into sections, at least three clamping bead sections being formed which reduce the diameter of the instrument opening (20).

7. Kit according to one of Claims 1 and 2, **characterized in that** the clamp element (23) is designed as a groove extending about a complete circumference or as a groove divided into sections.

8. Kit according to one of the preceding claims, **characterized in that** the seal (2) comprises a circumferential flange (26) on its outer face.

9. Kit according to one of the preceding claims, **characterized in that** the receiving opening (15) comprises a circumferential groove (16) in which a circumferential flange (26) of the seal (2) engages in order to establish a form-fit connection between trocar (1) and seal (2).

10. Kit according to one of the preceding claims, **characterized in that** the seal (2) is injected directly into the receiving opening (15) of the trocar (1) by a plastics injection-moulding technique.

11. Kit according to one of the claims 1 to 9, **characterized in that** the trocar (1) and the seal (2) are produced in one piece from a plastic.

12. Kit according to one of the preceding claims, **characterized in that** the complementary section has the shape of a groove which extends about a complete or partial circumference and into which the bead of the clamp element (23) engages.

13. Kit according to one of the preceding claims, **characterized in that** the complementary section has the shape of a bead which extends about a complete or partial circumference and which can be received by a groove arranged on the clamp element (23).

## Revendications

1. Ensemble comprenant un trocart (1) doté d'un joint d'étanchéité (2) et d'un instrument (3) configuré pour l'ophtalmologie,
le trocart (1) comprenant une canule (10) dotée d'un axe central (19), une bride (11) formée sur cette canule (10), une ouverture (12) qui traverse la canule (10) le long de l'axe central (19) et une ouverture de réception (15) qui traverse la bride (11) le long de l'axe central (19),
l'ouverture (12) et l'ouverture de réception (15) constituant une ouverture qui traverse complètement le trocart,
le joint d'étanchéité (2) étant disposé dans l'ouverture de réception (15) du trocart (1),
le joint d'étanchéité (2) comprenant un élément d'étanchéité (21) doté d'une ouverture d'étanchéité (22) et d'une ouverture d'instrument (20),
l'ouverture d'instrument (20) traversant le joint d'étanchéité (2) le long d'un axe central (29) sur les deux côtés de l'élément d'étanchéité (21),
**caractérisé en ce que**
le joint d'étanchéité (2) comporte en outre un élément de serrage (23) qui établit une liaison en correspondance géométrique avec un instrument (3) inséré au moins en partie dans l'ouverture d'instrument (20) et à travers l'ouverture d'étanchéité (22),
**en ce que** l'instrument (3) configuré pour l'ophtalmologie et qui peut être amené à traverser le trocart (1) comporte une partie complémentaire ou congruente de l'élément de serrage (23),
**en ce que** l'élément de serrage (23) et/ou l'instrument (3) sont configurés de telle sorte qu'ils peuvent être comprimés dans le joint d'étanchéité (2) pendant l'insertion de l'instrument (3), que ces pièces peuvent reprendre leur lorsqu'une profondeur d'insertion prédéfinie est atteinte, que la surface de l'élément de serrage (23) est en contact avec celle de la partie complémentaire de l'instrument et qu'une liaison en correspondance géométrique est établie entre le joint d'étanchéité (2) et l'instrument (3).

2. Ensemble selon la revendication 1, **caractérisé en ce que** l'élément de serrage (23) est formé d'un seul tenant avec le joint d'étanchéité (2).

3. Ensemble selon les revendications 1 ou 2, **caractérisé en ce que** l'élément d'étanchéité est une membrane (21) qui s'étend essentiellement à la perpendiculaire de l'axe central (29) du joint d'étanchéité (2), l'ouverture d'étanchéité (22) comportant au moins une fente.

4. Ensemble selon l'une des revendications précédentes, **caractérisé en ce que** l'élément de serrage (23) est disposé en avant de l'élément d'étanchéité (21) dans la direction d'insertion d'un instrument.

5. Ensemble selon l'une des revendications précédentes, **caractérisé en ce que** l'élément de serrage (23) est configuré comme bourrelet périphérique de serrage qui diminue le diamètre de l'ouverture d'instrument (20).

6. Ensemble selon l'une des revendications précédentes, **caractérisé en ce que** l'élément de serrage (23) est configuré comme bourrelet de serrage interrompu par parties, au moins trois parties de bourrelet de serrage qui diminuent le diamètre de l'ouverture d'instrument (20) étant formées.

7. Ensemble selon l'une des revendications 1 ou 2, **caractérisé en ce que** l'élément de serrage (23) est configuré comme rainure périphérique complète ou interrompue par parties.

8. Ensemble selon l'une des revendications précédentes, **caractérisé en ce que** le joint d'étanchéité (2) comporte une bride périphérique (26) sur son côté extérieur.

9. Ensemble selon l'une des revendications précédentes, **caractérisé en ce que** l'ouverture de réception (15) comporte une rainure périphérique (16) dans laquelle une bride périphérique (26) du joint d'étanchéité (2) s'engage en vue d'établir une liaison en correspondance géométrique entre le trocart (1) et le joint d'étanchéité (2).

10. Ensemble selon l'une des revendications précédentes, **caractérisé en ce que** le joint d'étanchéité (2) est injecté directement dans l'ouverture de réception (15) du trocart (1) par une opération d'injection de matière synthétique.

11. Ensemble selon l'une des revendications 1 à 9, **caractérisé en ce que** le trocart (1) et le joint d'étanchéité (2) sont réalisés d'une seule pièce en matière synthétique.

12. Ensemble selon l'une des revendications précédentes, **caractérisé en ce que** la partie complémentaire de l'instrument (3) présente la forme d'une rainure périphérique complète ou partie dans laquelle le bourrelet de l'élément de serrage (23) pénètre.

13. Ensemble selon l'une des revendications précédentes, **caractérisé en ce que** la partie complémentaire de l'instrument (3)
présente la forme d'un bourrelet périphérique complet ou partiel qui peut être repris par une rainure disposée sur l'élément de serrage (23).
